(19) Europäisches Patentamt
European Patent Office
Office européen des brevets

(11) Publication number: **0 097 340**
**B1**

(12) **EUROPEAN PATENT SPECIFICATION**

(45) Date of publication of patent specification: **15.10.86**

(21) Application number: **83105938.1**

(22) Date of filing: **16.06.83**

(51) Int. Cl.[4]: **C 07 D 295/02,**
C 07 D 295/06,
C 07 D 295/14,
C 07 D 295/12,
C 07 D 333/22,
C 07 D 317/28,
C 07 D 295/10,
C 07 D 263/20, C 07 D 263/28

(54) **Piperazine derivatives, a process for preparing them and pharmaceutical compositions.**

(30) Priority: **17.06.82 ES 514340**

(43) Date of publication of application:
**04.01.84 Bulletin 84/01**

(45) Publication of the grant of the patent:
**15.10.86 Bulletin 86/42**

(84) Designated Contracting States:
**BE CH DE FR GB IT LI NL**

(56) References cited:
**EP-A-0 050 072**
**DE-A-2 714 437**

**Chemical Abstracts vol. 86, no. 21, 23 May 1977, Columbus, Ohio, USA, R. NIKOLOV "Pharmalogical studies on derivatives of benzhydrylpiperazine", page 19, column 1, abstract no. 150351e**

**Chemical Abstracts vol. 82, no. 23, 9 June 1975, Columbus, Ohio, USA, page 61, column 1, abstract no. 156367d**

(73) Proprietor: **FERRER INTERNACIONAL, S.A.**
**Gran Via Carlos III, 94**
**08028 Barcelona (ES)**

(72) Inventor: **Foguet, Rafael**
**Llusanés 8**
**Barcelona (ES)**
Inventor: **Ortiz, José A.**
**Córcega 429**
**Barcelona (ES)**
Inventor: **Gubert, Santiago**
**Balmes 373**
**Barcelona (ES)**
Inventor: **Raga, Manuel M.**
**Sors 17-20**
**Barcelona (ES)**
Inventor: **Sacristán, Aurelio**
**Santa Amelia 2**
**Barcelona (ES)**

(74) Representative: **Kinzebach, Werner, Dr.**
**Patentanwälte Reitstötter, Kinzebach und Partner Sternwartstrasse 4 Postfach 86 06 49**
**D-8000 München 86 (DE)**

(56) References cited:
**Chemical Abstracts vol. 94, no. 13, 30 March 1981, Columbus, Ohio, USA, S. JUNG et al. "Preparation of cinnarizine", page 740, column 1, abstract no. 103301u**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 097 340 B1

## Description

The present invention relates to piperazine derivatives having the general formula (I):

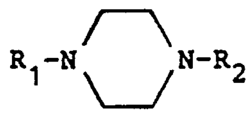

(I)

wherein:

$R_1$ is benzhydryl or cinnamyl and

$R_2$ is

A) a radical of the general formula (a)

$$-CH_2-CR_3=CHR_4 \quad (a)$$

wherein $R_3$ is hydrogen, chloromethyl, (4-benzhydryl-1-piperazinyl)-methyl, 4-morpholinylmethyl or 1-piperidinylmethyl and $R_4$ is hydrogen, chloromethyl or carbethoxy,

B) a radical of the formula (b)

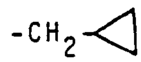

(b)

C) a radical of the general formula (c)

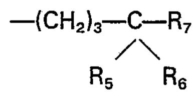

(c)

wherein $R_5$ and $R_6$ together represent an oxygen atom or $O-(CH_2)_2-O$ and $R_7$ is $C_1-C_6$-alkyl, preferably methyl, phenyl or 2-thienyl,

D) a radical of the general formula (d)

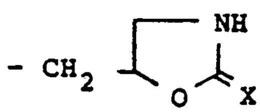

(d)

wherein X is O or NH,

E) a radical of the general formula (e)

$$-(CH_2)_3-R_8 \quad (e)$$

wherein $R_8$ is 4-morpholinyl, 1-piperidinyl or 4-benzhydryl-1-piperazinyl,

F) a radical of the general formula (f)

$$-CH_2NHR_9 \quad (f)$$

wherein $R_9$ is 2-oxo-1-(pyrrolidinyl)acetyl, 2-hydroxybenzoyl or 4-sulfamoylbenzoyl,

G) a radical of the formula (g)

$$-CH_2NH_2 \quad (g)$$

or

H) a radical of the general formula (h)

$$-COR_{10} \quad (h)$$

wherein $R_{10}$ is 2-oxo-1-(pyrrolidinyl)methyl or 4-sulfamoylphenyl, and the non-toxic addition salts thereof, as well as to a process for preparing these compounds and to pharmaceutical compositions containing these compounds. The process for preparing the compounds according to the invention is characterized in that

A) a monoalkylpiperazine of the general formula (II)

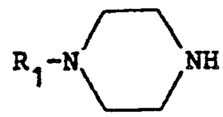

(II)

wherein $R_1$ is as defined in claim 1, is reacted with a compound of the general formula (III)

$$Y-R_{11} \qquad (III)$$

wherein Y is a chlorine, bromine or iodine atom and $R_{11}$ is a radical of the general formulae (a), (b), (c), (d) or (e) as defined in claim 1, to obtain a compound of the general formula I, wherein $R_1$ is as defined above and $R_2$ is a radical of the general formulae (a), (b), (c), (d) or (e), or a compound of the general formula (I), wherein $R_1$ is as defined above and $R_2$ is a radical of the formula

$—CH_2—C=CH_2$
|
$CH_2Cl$

is reacted with N-monobenzhydrylpiperazine, morpholine or piperidine, to obtain a compound of the general formula I, wherein $R_1$ is as defined above and $R_2$ is a radical of the general formula (a) wherein $R_3$ is (4-benzhydryl-1-piperazinyl)methyl, 4-morpholinylmethyl or 1-piperidinylmethyl and $R_4$ is hydrogen, or

B) a monoalkylpiperazine of the general formula II as defined above is reacted with a compound of the general formula IV

$$R_9NH_2 \qquad (IV)$$

wherein $R_9$ is as defined in claim 1, in the presence of formaldehyde to obtain a compound of the general formula (I) wherein $R_2$ is a radical of the general formula (f) as defined in claim 1, and if desired, the so obtained compound is hydrolyzed in acidic medium to a compound of the general formula (i) wherein $R_2$ is a radical of the formula (g) as defined in claim 1, and if desired, the so obtained compound is reacted with a carboxylic acid of the general formula V

$$R_9OH \qquad (V)$$

wherein $R_9$ is as defined in claim 1, to obtain a compound of the general formula (I), wherein $R_2$ is a radical of the general formula (f), or

C) a monoalkylpiperazine of the general formula (II) as defined above is reacted with a carboxylic acid of the general formula (VI)

$$R_{10}COOH$$

wherein $R_{10}$ is as defined in claim 1, to obtain a compound of the general formula (I) wherein $R_2$ is a radical of the general formula (b) as defined in claim 1, and if desired, the so obtained piperazine compounds are converted to their non-toxic addition salts.

The reaction of the monoalkylpiperazine of the general formula (II) with a compound of the general formula (III) is preferably conducted in an alkanol having 1 to 4 carbon atoms and in the presence of a base selected from the group consisting of an alkali metal or alkaline earth metal carbonate or bicarbonate and a tertiary amine.

Suitable bases are sodium, potassium, magnesium or calcium carbonate or bicarbonate or trimethylamine, tri-n-propylamine, tri-n-butylamine or pyridine. The preferred base is sodium bicarbonate or triethylamine.

Preferably the process is carried out at elevated temperatures, most preferably at reflux temperatures.

The alkylation of N-monobenzhydrylpiperazine, morpholine or piperidine with the compounds of the general formula (I), wherein $R_1$ is as defined above and $R_2$ is a radical of the general formula (a) ($R_3$ = chloromethyl; $R_4$ = hydrogen) is preferably performed in an alkanol having 1 to 4 carbon atoms at elevated temperatures thus obtaining piperazine derivatives of the general formula (I) wherein $R_2$ is a radical of the general formula (a) ($R_3$ = (4-benzhydryl-1-piperazinyl)-methyl, 4-morpholinylmethyl or 1-piperidinyl-methyl; $R_4$ = hydrogen).

Most preferably the reaction is carried out at reflux temperatures.

When a monoalkyl piperazine of the general formula (II) is reacted with an amide of the general formula (IV) in the presence of formaldehyde it is preferred to use an alkanol having 1 to 4 carbon atoms as solvent, preferably ethanol, at elevated temperatures, preferably under reflux.

Hydrolysis of the so obtained compounds in an acidic medium leads to cleavage of the carboxamido group to obtain piperazine derivatives wherein $R_2$ is aminomethyl [formula (g)]. This reaction is preferably carried out in a mixture of acetic acid and hydrochloric acid at elevated temperatures, preferably under reflux.

Reaction of the compounds of the general formula (I) wherein $R_2$ is aminomethyl with carboxylic acids of the general formula (V) is preferably carried out in the presence of a catalyst in N,N-dimethylformamide or an ether, preferably tetrahydrofurane, or a mixture thereof at room temperature to obtain compounds of the general formula (I), wherein $R_2$ is a radical of the general formula (f). The preferred catalyst is N,N-carbonyldiimidazole.

For reaction of a monoalkylpiperazine of the general formula (II) with a carboxylic acid of general formula (VI), preferably a catalyst and N,N-dimethylformamide or an ether, preferably tetrahydrofurane, or a mixture thereof as a solvent is used.

The preferred catalyst is N,N-carbonyldiimidazole.

The compounds of the present invention have cardiovascular properties. Structurally related compounds having also cardiovascular properties are known from C.A. 86:150351e and EP—A—50072. In this context it is emphasized that the compounds of the invention are able to remarkably increase the cerebral flow as experimentally determined by an electromagnetic flowmeter. The results of the cardiovascular evaluation of the most active compounds of the invention in comparison to the results obtained with Aligeron and Cinnarizine are shown in Tables 1(i) and 1(ii).

TABLE 1(i)

| Parameters / Compounds | Antiarrhythmic activity $ED_{50}$ (mg/kg) — 1 — | Mean arterial pressure, Hypotension $ED_{50}$ (mg/kg) — 2 — | Heart rate, Bradycardia $ED_{50}$ (mg/kg) — 3 — |
|---|---|---|---|
| 1 | 4.2 | 2.7 | 3.4 |
| 6 | 4.3 | 1.5 | 1.7 |
| 7 | 23.0 | 2.1 | 3.8 |
| 11 | 6.9 | 0.43 | 2.1 |
| 13 | 4.0 | 1.5 | 2.6 |
| 15 | 16.5 | 1.2 | 3.0 |
| Cinnarizine 2HCl | 23.0 | 1.0 | 2.8 |
| Aligeron-2HCl *) | 4.4 | 1.3 | 4.8 |

*) Said compound is known from C.A. 86:150351e see above

TABLE 1(ii)

| Parameters / Compounds | Cerebral flow Increase (%) — 4 — | Carotid flow Decrease (%) — 5 — | Arterial pressure Decrease (%) — 6 — | Cerebr. vasc. resistance Decrease (%) — 7 — |
|---|---|---|---|---|
| 1 | 55.0 | 68.0 | 30.0 | 55.0 |
| 6 | 70.0 | 22.0 | 58.0 | 75.3 |
| 7 | 32.0 | 13.0 | 27.3 | 44.7 |
| 11 | 81.0 | 40.9 | 50.0 | 74.0 |
| 13 | 140.1 | 25.7 | 35.0 | 73.1 |
| 15 | 38.5 | 60.0 | 45.5 | 60.3 |
| Cinnarizine 2HCl | 76.7 | 62.8 | 43.0 | 68.0 |
| Aligeron 2HCl | 64.0 | 21.3 | 36.0 | 61.0 |

The antiarrhythmic activity (Parameter 1) has been measured according to the method by Ferrini et al Arzn,-Forsch., 29(II), 9a, 1947—77, 1979). The compounds were endovenously administered to mice and the results are expressed as $ED_{50}$-values.

The decrease in arterial pressure (Parameter 2) and the effects on the heart rate (Parameter 3) have been evaluated according to usual methods in Cardiovascular Pharmacology. The compounds were endovenously administered to anesthetized rats and results have been tabulated as $ED_{50}$ values.

However, measurements of cerebral and carotid flow changes (Parameters 4 and 5) have been made by most sophisticated methods. Both parameters have been determined by electromagnetic flowmetry (Narcomatic-Electromagnetic Flowmeter) on 12 anesthetized dogs. The hemodynamic parameter average of animals before testing were $pO_2$ = 95.40 ± 3.98; $pCO_2$ = 34.31 ± 2.38 and pH = 7.297 ± 0.315 by endovenously administering the compounds at a 5 mg/kg dosis.

Simultaneously to the measuring of cerebral and carotid flows, the mean arterial pressure and cerebral vascular resistance (Parameters 6 and 7) in each animal was determined. These four parameters were measured according to the usual method in Cardiovascular Pharmacology and the values were tabulated as the variation rate (increase or decrease) over mean values before testing.

The toxicity of some compounds is lower than the toxicity of Aligeron and Cinnarizine. Therefore the present compounds are very useful in therapy. $LD_{50}$ was endovenously determined in mice according to the Reed-Münch's method as modified by Pizzi (Human Biology, 22(3), 151—190, 1950) and the results are comparatively shown versus Aligeron and Cinnarizine in Table 2.

TABLE 2

| Compounds | Acute toxicity $LD_{50}$ (mg/kg) |
|---|---|
| 1 | 25.6 ± 4.39 |
| 6 | 8.6 ± 0.54 |
| 7 | 100.0 ± 6.27 |
| 11 | 21.0 ± 1.72 |
| 13 | 42.5 ± 3.05 |
| 15 | 40.0 ± 4.19 |
| Cinnarizine 2HCl | 47.6 ± 5.25 |
| Aligeron 2HCl | 38.5 ± 1.06 |

The compounds of the present invention mixed with pharmaceutically acceptable carriers can be administered by the oral route in form of tablets, capsules, syrup, solutions, etc., by injectable route and by rectal route, at daily doses ranging from 50 to 500 mg.

Example 1

1-Benzhydryl-4-cyclopropylmethyl-piperazine dihydrochloride (Compound 1)
(I, $R_1$ = benzhydryl, $R_2$ = cyclopropylmethyl)

14,0 g of N-monobenzhydryl-piperazine (0,055 mole) are dissolved in 100 ml of absolute ethanol in the presence of 6.5 g of sodium bicarbonate (0.077 mole). The resulting mixture is gently treated with 6,03 g solution of chloromethylcyclopropane (0,066 mole) in 10 ml of absolute ethanol, then it is subjected to reflux for 20 hours. After conditioning the reaction mass to room temperature, it is then filtered off and the ethanol is removed by distillation. The resulting crude material is dissolved in 100 ml of 3N hydrochlorid acid, washed with ethyl ether and extracted with chloroform. The organic extracts are dried over anhydrous magnesium sulphate, filtered and the solvent is removed by distillation. The resulting crude material is recrystallized from 96% ethanol, thus obtaining 11,5 g of crystals of 1-benzhydryl-4-cyclopropylmethyl-piperazine dihydrochloride (I, $R_1$ = benzhydryl, $R_2$ = cyclopropylmethyl).
Yield: 55%, m.p. 245—247°C (d);
IR (KBr), $cm^{-1}$: 3000—2900, 2800—2400, 770, 755, 715, 705.

Example 2

According to the process described in Example 1 and using respective starting intermediates, the compounds listed in Table 3 are obtained.

TABLE 3

| Compounds | Compound (I) R1 | Compound (I) R2 | Base or Salt | M.P. °C |
|---|---|---|---|---|
| 2 | $(C_6H_5)_2CH-$ | $-CH_2-CH=CH-CH_2-Cl$ | Base<br>2HCl | 174–176<br>215–221 |
| 3 | $(C_6H_5)_2CH-$ | 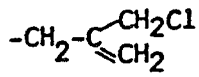 | Base | 77,5–77,9 |
| 4 | $(C_6H_5)_2CH-$ | 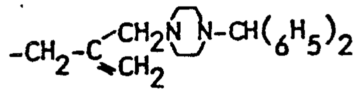 | Base | 139–142 |
| 5 | $(C_6H_5)_2CH-$ | 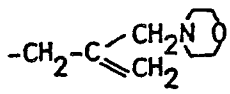 | Base | 167–171 |
| 6 | $(C_6H_5)_2CH-$ | 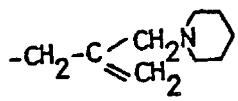 | Base | 99–102 |
| 7 | $(C_6H_5)_2CH-$ | $-CH_2-CH=CH-COO_2H_5$ | 2HCl | 217,5–218 |
| 8 | $C_6H_5-CH=CH-CH_2-$ | 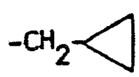 | 2HCl | >250 (d) |
| 9 | $(C_6H_5)_2CH-$ | 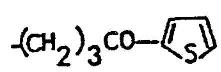 | Base | 105–108 |
| 10 | $(C_6H_5)_2CH-$ | 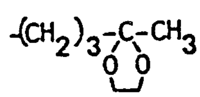 | Base | 73–75 |
| 11 | $(C_6H_5)_2CH-$ | 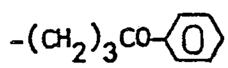 | 2HCl | 209–212 |
| 12 | $(C_6H_5)_2CH-$ | $-(CH_2)_3COCH_3$ | 2HCl | 211–212 |
| 13 | $(C_6H_5)_2CH-$ | 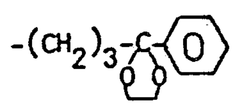 | Base | 93–97 |
| 14 | $(C_6H_5)_2CH$ | 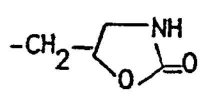 | 2HCl | 122–130 |

TABLE 3 (Continued)

| Compounds | Compound (I) $R_1$ | $R_2$ | Base or Salt | M.P. °C |
|---|---|---|---|---|
| 15 | $(C_6H_5)_2CH$ | 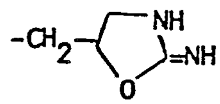 | 2HCl | 180—190 |
| 16 | $C_6H_5-CH=CH-CH_2-$ | 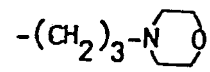 | 3HCl | >250 (d) |
| 17 | $C_6H_5-CH=CH-CH_2-$ | 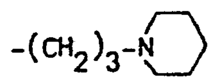 | 3HCl | >250 (d) |
| 18 | $(C_6H_5)_2CH-$ | 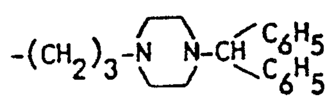 | 4HCl | 240—2 (d) |

## Example 3

a) 1-Benzhydryl-4-[2-(1-piperidinylmethyl)-2-propenyl]piperazine (Compound 6)
(I, $R_1$ = benzhydryl, $R_2$ = 2-(1-piperidinylmethyl)-2-propenyl)
from 1-benzhydryl-4-(2-chloromethyl-2-propenyl)piperazine (Compound 3)
(I, $R_1$ = benzhydryl, $R_2$ = 2-chloromethyl-2-propenyl)

A mixture of 7,0 g of 1-benzhydryl-4-(2-chloromethyl-2-propenyl)-piperazine (0.0205 mole) and 6.98 g of piperidine (0.082 mole) in 150 ml of absolute ethanol is heated until a solution is obtained, and subjected to reflux for 3 hours. Then, it is conditioned to room temperature and the ethanol is removed by distillation. The resulting crude material is several times treated with distilled water until a solid is obtained, which is then filtered and washed abundantly with water. By recrystallization from ethanol, 7,4 g crystals of 1-benzhydryl-4-[2-(1-piperidinylmethyl)-2-propenyl]-piperazine (I, $R_1$ = benzhydryl, $R_2$ = 2-(1-piperidinylmethyl-2-propenyl) are obtained.
Yield: 94%; m.p. 99—102°C.

b) 1-Benzhydryl-4-(2-[(4-benzhydryl-1-piperazinyl)methyl]-2-propenyl)-piperazine (Compound 4)
(I, $R_1$ = benzhydryl, $R_2$ = 2-[(4-benzhydryl-1-piperazinyl)methyl]-2-propenyl)
from 1-benzhydryl-4-(2-chloromethyl-2-propenyl)-piperazine (Compound 3)
(I, $R_1$ = benzhydryl, $R_2$ = 2-chloromethyl-2-propenyl)

According to the process described above in a) using N-monobenzhydryl-piperazine instead of piperidine, Compound 4 is obtained; m.p. 139—142°C.

c) 1-Benzhydryl-4-[2-(4-morpholinylmethyl)-2-propenyl]-piperazine (Compound 5)
(I, $R_1$ = benzhydryl, $R_2$ = 2-(4-morpholinylmethyl)-2-propenyl
from 1-benzhydryl-4-(2-chloromethyl-2-propenyl-piperazine (Compound 3)
(I, $R_1$ = benzhydryl, $R_2$ = 2-chloromethyl-2-propenyl).

According to the process described above in (a) and using morpholine instead of piperidine, Compound 5 is obtained; m.p. 167—171°C.

## Example 4

1-Benzhydryl-4-(2-oxo-1-pyrrolidinyl)acetyl)methylpiperazine (Compound 19)
(I, $R_1$ = benzhydryl, $R_2$ = [2-oxo-1-(pyrrolidinyl)acetyl]methyl)

5,04 g of N-monobenzhydryl-piperazine (0,02 mole) and 2,84 g of 2-oxo-1-pyrrolidinylacetamide (0,02 mole) are dissolved in 100 ml of absolute ethanol, then 2,6 ml of 35% (p/v) formaldehyde solution is added and the mixture subjected to reflux for 12 hours. The reaction mixture is allowed to stand with stirring for 20 hours at room temperature. The ethanol is removed by distillation and the resulting oil is treated with 60 ml of ethyl ether for 1 hour, thus yielding a crystal-like solid which is filtered, washed with ethyl ether and

dried under vacuum. 6,9 g crystals of 1-benzhydryl-4-[2-oxo-1-(pyrrolidinyl)acetyl]-methyl-piperazine, (I, $R_1$ = benzhydryl, $R_2$ = [2-oxo-1-(pyrrolidinyl)acetyl)methyl) are obtained.
Yield: 85%; m.p. 157—158,5°C;
IR (KBr), $cm^{-1}$: 3460, 3220, 1705, 1680, 1545.

Example 5

As described in Example 4 using respective starting intermediates, the compounds listed below (Table 4) are obtained.

TABLE 4

| Compounds | Compound (I) $R_1$ | $R_2$ | Salt | M.P. °C |
|---|---|---|---|---|
| 20 | $(C_6H_5)_2CH-$ | 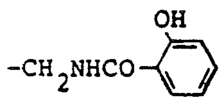 | 2HCl | 158–162 |
| 21 | $(C_6H_5)_2CH-$ | 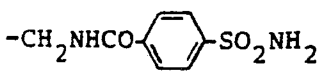 | HCl | 265–6 (d) |

Example 6

1-Benzhydryl-4-aminomethyl-piperazine trihydrochloride (Compound 22)
(I, $R_1$ = benzhydryl, $R_2$ = aminomethyl)

3.80 g of 1-benzhydryl-4-[2-oxo-1-(pyrrolidinyl)acetyl]-methyl-piperazine (0,0093 mole) are treated with 20 ml of glacial acetic acid and 40 ml of 3N hydrochloric acid, and subjected to reflux for 2 hours. Once the reaction is completed, the solvents are removed by distillation under vacuum. The resulting crude product is treated with 100 ml of acetone thus yielding a crystalline solid which is dried under vacuum. 2.05 g crystals of 1-benzhydryl-4-aminomethyl-piperazine trihydrochloride (I, $R_1$ = benzhydryl, $R_2$ = aminomethyl).
Yield: 56%, m.p. >250°C (d) are obtained.
IR (KBr), $cm^{-1}$: 1590, 1400, 750, 710.

Example 7

1-Benzhydryl-4-[(4-sulfamoylbenzoyl)amino]-methyl-piperazine hydrochloride (Compound 21)
(I, $R_1$ = benzhydryl, $R_2$ = [(4-sulfamoylbenzoyl)amino]-methyl).

2.01 g of 4-sulfamoylbenzoic acid (0,01 mole) and 1.62 g of carbonyldiimidazol (0,01 mole) are dissolved in 40 ml of N,N-dimethylformamide. The mixture is allowed to stand with stirring for 4 hours at room temperature and then 2,81 g of 1-benzhydryl-4-aminomethyl-piperazine (0,01 mole) in 40 ml of dry tetrahydrofurane are added.

The resulting mixture is allowed to stand with stirring for 3 hours at room temperature. The reaction liquid is treated with 100 ml of ethyl acetate and 100 ml of distilled water. Both phases are separated and the organic phase is washed with water. The ethyl acetate is removed by distillation and the resulting solid is treated with 100 ml of ethyl ether, filtered and washed with ether, then dissolved in ethanol and finally 20 ml of saturated hydrogen chloride are added, thus obtaining 2,35 g crystals of 1-benzhydryl-4-[(4-sulfamoyl-benzoyl)amino]methyl-piperazine hydrochloride, [I, $R_1$ = benzhydryl, $R_2$ = [(sulfamoyl-benzoyl)-amino)methyl].
Yield: 47%, m.p. 265—266°C (d).
IR (KBr), $cm^{-1}$: 3240, 1630, 1345, 1160.

Example 8

1-Benzhydryl-4-(4-sulfamoylbenzoyl)piperazine (Compound 23)
(I, $R_1$ = benzhydryl, $R_2$ = 4-sulfamoylbenzoyl)

20,01 g of 4-sulfamoylbenzoic acid (0,01 mole) and 1,62 g of carbonyldiimidazol (0,01 mole) are dissolved in 40 ml of anhydrous N,N-dimethylformamide. The mixture is allowed to stand with stirring for 3 hours at room temperature and then 2,52 g of N-monobenzhydryl-piperazine (0,01 mole) are added. The solution thus obtained is allowed to stand with stirring for 24 hours at room temperature. Then, 100 ml of

distilled water and 100 ml of ethyl acetate are added; both phases are separated and the organic phase is washed with distilled water, then dried over anhydrous magnesium sulphate, filtered and the ethyl acetate is removed by distillation under vacuum. The resulting crude material is treated with ethyl ether and 2,9 g crystals of 1-benzhydryl-4-(4-sulfamoylbenzoyl)piperazine (I, $R_1$ = benzhydryl, $R_2$ = 4-sulfamoylbenzoyl) are obtained.
Yield: 67%, m.p. 228—231°C.
IR (KBr), $cm^{-1}$: 3260, 3400, 1630, 1170, 1345.

Example 9

1-Benzhydryl-4-[2-oxo-1-(pyrrolidinyl)acetyl]-piperazine (Compound 24)
(I, $R_1$ = benzhydryl, $R_2$ = 2-oxo-1-(pyrrolidinyl)acetyl)

As described in Example 8 using 2-oxo-1-(pyrrolidinyl)acetic acid instead of 4-sulfamoylbenzoic acid, Compound 24 is obtained, m.p. 169—170°C, Dihydrochloride, m.p. 204—206°C.

**Claims**

1. Piperazine derivatives having the general Formula (I):

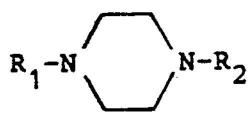

(I)

wherein:
$R_1$ is benzhydryl or cinnamyl and
$R_2$ is
A) a radical of the general formula (a)

$$—CH_2—CR_3=CHR_4 \quad (a)$$

wherein $R_3$ is hydrogen, chloromethyl, (4-benzhydryl-1-piperazinyl)-methyl, 4-morpholinylmethyl or 1-piperidinylmethyl and $R_4$ is hydrogen, chloromethyl or carbethoxy,
B) a radical of the formula (b)

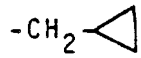

(b)

C) a radical of the general formula (c)

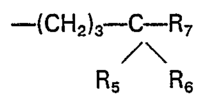

(c)

wherein $R_5$ and $R_6$ together represent an oxygen atom or $O—(CH_2)_2—O$ and $R_7$ is $C_1—C_6$-alkyl, phenyl or 2-thienyl,
D) a radical of the general formula (d)

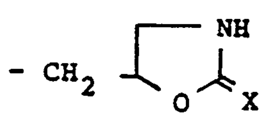

(d)

wherein X is O or NH,
E) a radical of the general formula (e)

$$—(CH_2)_3—R_8 \quad (e)$$

wherein $R_8$ is 4-morpholinyl, 1-piperidinyl or 4-benzhydryl-1-piperazinyl,
F) a radical of the general formula (f)

$$—CH_2NHR_9 \quad (f)$$

wherein $R_9$ is 2-oxo-1-(pyrrolidinyl)acetyl, 2-hydroxybenzoyl or 4-sulfamoylbenzoyl,
G) a radical of the formula (g)

$$—CH_2NH_2 \quad (g)$$

or

H) a radical of the general formula (h)

$$-COR_{10} \qquad (h)$$

wherein $R_{10}$ is 2-oxo-1-(pyrrolidinyl)methyl or 4-sulfamoylphenyl, and the non-toxic addition salts thereof.

2. A process for preparing the compounds according to claim 1, characterized in that

A) a monoalkylpiperazine of the general formula (II)

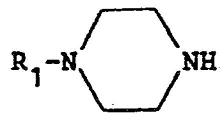

(II)

wherein $R_1$ is as defined in claim 1, is reacted with a compound of the general formula (III)

$$Y-R_{11} \qquad (III)$$

wherein Y is a chlorine, bromine or iodine atom and $R_{11}$ is a radical of the general formulae (a), (b), (c), (d) or (e) as defined in claim 1, to obtain a compound of the general formula I, wherein $R_1$ is as defined above and $R_2$ is a radical of the general formulae (a), (b), (c), (d) or (e), or a compound of the general formula (I), wherein $R_1$ is as defined above and $R_2$ is a radical of the formula

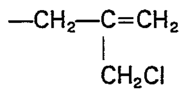

is reacted with N-monobenzhydrylpiperazine, morpholine or piperidine, to obtain a compound of the general formula I, wherein $R_1$ is as defined above and $R_2$ is a radical of the general formula (a) wherein $R_3$ is (4-benzhydryl-1-piperazinyl)methyl, 4-morpholinylmethyl or 1-piperidinylmethyl and $R_4$ is hydrogen, or

B) a monoalkylpiperazine of the general formula II is reacted with a compound of the general formula IV

$$R_9NH_2 \qquad (IV)$$

wherein $R_9$ is as defined in claim 1, in the presence of formaldehyde to obtain a compound of the general formula (I) wherein $R_2$ is a radical of the general formula (f) as defined in claim 1, and if desired, the so obtained compound is hydrolyzed in acidic medium to a compound of the general formula (I) wherein $R_2$ is a radical of the formula (g) as defined in claim 1, and if desired, the so obtained compound is reacted with a carboxylic acid of the general formula V

$$R_9OH \qquad (V)$$

wherein $R_9$ is as defined in claim 1, to obtain a compound of the general formula (I), wherein $R_2$ is a radical of the general formula (f), or

C) a monoalkylpiperazine of the general formula (II) is reacted with a carboxylic acid of the general formula (VI)

$$R_{10}COOH$$

wherein $R_{10}$ is as defined in claim 1, to obtain a compound of the general formula (I) wherein $R_2$ is a radical of the general formula (h) as defined in claim 1, and, if desired, the so obtained piperazine compounds are converted to their non-toxic addition salts.

3. A process according to claim 2, characterized in that the reactions according to A) and B) are carried out in a $C_1$—$C_4$-alkanol at elevated temperatures and in the presence of an alkali metal or alkaline earth metal carbonate or bicarbonate or a tertiary amine.

4. A process according to claim 3, characterized in that the reaction is carried out in ethanol at reflux temperature in the presence of sodium bicarbonate or triethylamine.

5. A process according to claim 2, characterized in that the hydrolysis of the compound of the general formula (I) wherein $R_2$ is a radical of the general formula (f) is carried out in an aqueous solution of a mixture of acetic acid and hydrochloric acid at elevated temperatures, preferably at reflux temperature.

6. A process according to claim 2, characterized in that the reaction of a compound of the general formula (I) wherein $R_2$ is a radical of the formula (g) with a carboxylic acid of the general formula (V) is carried out in an ether-like solvent, preferably tetrahydrofurane, N,N-dimethylformamide or a mixture thereof, in the presence of a catalyst, preferably carbonyldiimidazole.

7. A process according to claim 2, characterized in that the reaction of a compound of the general

formula (II) with a carboxylic acid of the general formula (VI) is carried out in an ether-like solvent, preferably tetrahydrofurane, N,N-dimethylformamide or a mixture thereof, in the presence of a catalyst, preferably carbonyldiimidazole.

8. A process according to claim 2, characterized in that the conversion of the piperazine compounds to their non-toxic addition salts is carried out in a $C_1$—$C_4$ alkanol, preferably methanol, ethanol or isopropanol.

9. Pharmaceutical composition comprising at least one compound according to claim 1, optionally in combination with pharmaceutically acceptable carriers and adjuvants.

**Patentansprüche**

1. Piperazin-Derivate der allgemeinen Formel (I):

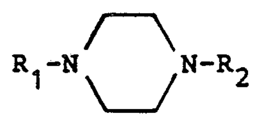

(I)

worin

$R_1$ für Benzhydryl oder Cinnamyl steht und

$R_2$ für

A) einen Rest der allgemeinen Formel (a)

$$—CH_2—CR_3{=}CHR_4 \qquad (a)$$

worin

$R_3$ Wasserstoff, Chlormethyl, (4-Benzhydryl-1-piperazinyl)-methyl, 4-Morpholinylmethyl oder 1-Piperidinylmethyl bedeutet, und

$R_4$ für Wasserstoff, Chlormethyl oder Carbethoxy steht,

B) einen Rest der Formel (b)

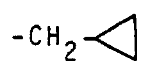

(b)

C) einen Rest der allgemeinen Formel (c)

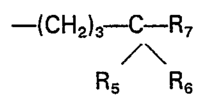

(c)

worin

$R_5$ und $R_6$ zusammen für einen Sauerstoffatom oder O—$(CH_2)_2$—O stehen, und

$R_7$ für $C_1$—$C_6$-Alkyl oder 2-Thienyl steht,

D) einen Rest der allgemeinen Formel (d)

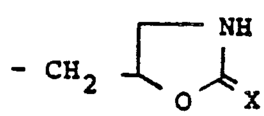

(d)

worin

X für O oder NH steht,

E) einen Rest der allgemeinen Formel (e)

$$—(CH_2)_3—R_8 \qquad (e)$$

worin

$R_8$ für 4-Morpholinyl, 1-Piperidinyl oder 4-Benzhydryl-1-piperazinyl steht,

F) einen Rest der allgemeinen Formel (f)

$$—CH_2NHR_9 \qquad (f)$$

worin

$R_9$ 2-Oxo-1-(pyrrolidinyl)acetyl, 2-Hydroxybenzoyl oder 4-Sulfamoylbenzoyl bedeutet,

G) einen Rest der Formel (g)

$$—CH_2NH_2 \qquad (g)$$

oder

H) einen Rest der allgemeinen Formel (h)

$$-COR_{10} \qquad (h)$$

worin

$R_{10}$ 2-Oxo-1-(pyrrolidinyl)methyl oder 4-Sulfamoylphenyl bedeutet, steht, und die nicht-toxischen Additionssalze davon.

2. Verfahren zur Herstellung der Verbindungen nach Anspruch 1, dadurch gekennzeichnet, daß man

A) ein Monoalkylpiperazin der allgemeinen Formel (II)

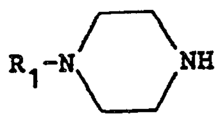

(II)

worin

$R_1$ die in Anspruch 1 angegebene Bedeutung besitzt, mit einer Verbindung der allgemeinen Formel (III)

$$Y-R_{11} \qquad (III)$$

worin

Y für ein Chlor-, Brom- oder Jodatom, und

$R_{11}$ für einen Rest der allgemeinen Formeln (a), (b), (c), (d) oder (e) gemäß Anspruch 1 stehen, zu einer Verbindung der allgemeinen Formel (I), worin

$R_1$ die oben angegebene Bedeutung besitzt, und

$R_2$ für einen Rest der allgemeinen Formeln (a), (b), (c), (d) oder (e) steht, umsetzt, oder eine Verbindung der allgemeinen Formel (I), worin

$R_1$ die oben angegebene Bedeutung besitzt, und

$R_2$ für einen Rest der Formel

$$-CH_2-C=CH_2$$
$$\quad\;|$$
$$\quad CH_2Cl$$

steht, mit N-Monobenzhydrylpiperazin, Morpholin oder Piperidin zu einer Verbindung der allgemeinen Formel (I), worin

$R_1$ die oben angegebene Bedeutung besitzt, und

$R_2$ für einen Rest der allgemeinen Formel (a) steht, worin

$R_3$ für (4-Benzhydryl-1-piperazinyl)methyl, 4-Morpholinylmethyl oder 1-Piperidinylmethyl, und

$R_4$ für Wasserstoff stehen, umsetzt, oder

B) ein wie oben definiertes Monoalkylpiperazin der allgemeinen Formel (II) mit einer Verbindung der allgemeinen Formel (IV)

$$R_9NH_2 \qquad (IV)$$

worin

$R_9$ die in Anspruch 1 angegebene Bedeutung besitzt, in Gegenwart von Formaldehyd zu einer Verbindung der allgemeinen Formel (I), worin $R_2$ für einen in Anspruch 1 definierten Rest der allgemeinen Formel (f) steht, umsetzt und gewünschtenfalls die so erhaltene Verbindung im sauren Medium zu einer Verbindung der allgemeinen Formel (I) hydrolysiert, worin $R_2$ für einen in Anspruch 1 definierten Rest der allgemeinen Formel (g) steht, und gewünschtenfalls die so erhaltene Verbindung mit einer Carbonsäure der allgemeinen Formel (V)

$$R_9OH \qquad (V)$$

worin

$R_9$ die in Anspruch 1 angegebene Bedeutung besitzt, zu einer Verbindung der allgemeinen Formel (I), worin $R_2$ für einen Rest der allgemeinen Formel (f) steht, umsetzt, oder

C) ein wie zuvor definiertes Monoalkylpiperazin der allgemeinen Formel (II) mit einer Carbonsäure der allgemeinen Formel (VI)

$$R_{10}COOH$$

worin

$R_{10}$ die in Anspruch 1 angegebene Bedeutung besitzt, zu einer Verbindung der allgemeinen Formel (I),

worin $R_2$ für einen wie in Anspruch 1 definierten Rest der allgemeinen Formel (h) steht, umsetzt und gewünschtenfalls die so erhaltenen Piperazin-Verbindungen in ihre nicht-toxischen Additionssalze überführt.

3. Verfahren nach Anspruch 2, dadurch gekennzeichnet, daß man die Umsetzungen gemäß A) und B) in einem $C_1$—$C_4$-Alkanol bei erhöhter Temperatur und in Gegenwart eines Alkalimetall- oder Erdalkalimetallcarbonats oder -bicarbonats oder eines tertiären Amins durchführt.

4. Verfahren nach Anspruch 3, dadurch gekennzeichnet, daß man die Umsetzung in Ethanol bei der Rückflußtemperatur in Gegenwart von Natriumbicarbonat oder Triethylamin durchführt.

5. Verfahren nach Anspruch 2, dadurch gekennzeichnet, daß man die Hydrolyse der Verbindung der allgemeinen Formel (I), worin $R_2$ für einen Rest der allgemeinen Formel (f) steht, in einer wässrigen Lösung einer Mischung aus Essigsäure und Salzsäure bei erhöhter Temperatur, vorzugsweise bei der Rückflußtemperatur, dirchführt.

6. Verfahren nach Anspruch 2, dadurch gekennzeichnet, daß man die Umsetzung einer Verbindung der allgemeinen Formel (I), worin $R_2$ für einen Rest der Formel (g) steht, mit einer Carbonsäure der allgemeinen Formel (V) in einem Ether-ähnlichen Lösungsmittel, vorzugsweise Tetrahydrofuran, N,N-Dimethylformamid oder einer Mischung davon, in Gegenwart eines Katalysators, vorzugsweise Carbonyldiimidazol, durchführt.

7. Verfahren nach Anspruch 2, dadurch gekennzeichnet, daß man die Umsetzung einer Verbindung der allgemeinen Formel (II) mit einer Carbonsäure der allgemeinen Formel (VI) in einem Ether-ähnlichen Lösungsmittel, vorzugsweise Tetrahydrofuran, N,N-Dimethylformamid oder einer Mischung davon, in der Gegenwart eines Katalysators, vorzugsweise Carbonyldiimidazol, durchführt.

8. Verfahren nach Anspruch 2, dadurch gekennzeichnet, daß man die Überführung der Piperazin-Verbindungen in ihre nicht-toxischen Additionssalze in einem $C_1$—$C_4$-Alkanol, vorzugsweise Methanol, Ethanol oder Isopropanol, durchführt.

9. Pharmazeutisches Mittel, welches mindestens eine eine Verbindung nach Anspruch 1 enthält, gegebenenfalls in Kombination mit pharmazeutisch verträglichen Trägern und Adjuvantien.

**Revendications**

1. Dérivés de la pipérazine, ayant la formule générale (I):

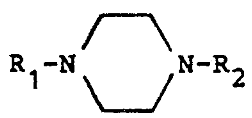

(I)

dans laquelle:

$R_1$ est un groupe benzhydryle ou cinnamyle, et

$R_2$ est

A) un radical de formule générale (a)

$$—CH_2—CR_3{=}CHR_4 \qquad (a)$$

dans laquelle $R_3$ est un atome d'hydrogène, ou un groupe chlorométhyle, (4-benzhydryl-1-pipérazinyl)-méthyle, 4-morpholinylméthyle ou 1-pipéridinylméthyle, et $R_4$ est un atome d'hydrogène, ou un groupe chlorométhyle ou carbéthoxy,

B) un radical de formule (b)

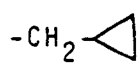

(b)

C) un radical de formule générale (c)

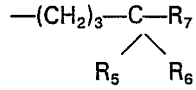

(c)

dans laquelle $R_5$ et $R_6$ représentent ensemble un atome d'oxygène ou un groupe $—O—(CH_2)_2—O—$, et $R_7$ est un groupe alkyle en $C_{1-6}$ phényle, ou 2-thiényle,

D) un radical de formule générale (d)

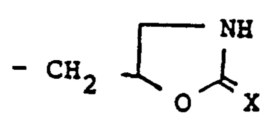

(d)

dans laquelle X est O ou NH,

E) un radical de formule générale (e)

$$-(CH_2)_3-R_8 \qquad (e)$$

dans laquelle $R_8$ est un groupe 4-morpholinyle, 1-pipéridinyle ou 4-benzhydryl-1-pipérazinyle,

F) un radical de formule générale (f)

$$-CH_2NHR_9 \qquad (f)$$

dans laquelle $R_9$ est un groupe 2-oxo-1-(pyrrolidinyl)acétyle, 2-hydroxybenzoyle, ou 4-sulfamoylbenzoyle,

G) un radical de formule (g)

$$-CH_2NH_2 \qquad (g)$$

ou

H) un radical de formule générale (h)

$$-COR_{10} \qquad (h)$$

dans laquelle $R_{10}$ est un groupe 2-oxo-1-(pyrrolidinyl)-méthyle ou 4-sulfamoylphényle, et les sels d'addition non toxiques de ces dérivés.

2. Procédé de préparation des composés conformes à la revendication 1, caractérisé en ce que:

A) on fait réagir une monoalkylpipérazine de formule générale (II)

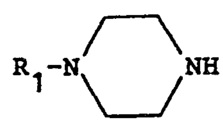

(II)

dans laquelle $R_1$ est tel que défini dans la revendication 1, avec un composé de formule générale (III)

$$Y-R_{11} \qquad (III)$$

dans laquelle Y est un atome de chlore, de brome, ou d'iode et $R_{11}$ est un radical de formule générale (a), (b), (c), (d) ou (e), telles que définies dans la revendication 1, pour obtenir un composé de formule générale I, dans laquelle $R_1$ est tel que défini ci-dessus et $R_2$ est un radical de formule générale (a), (b), (c), (d) ou (e), ou bien on fait réagir un composé de formule générale (I), dans laquelle $R_1$ est tel que défini ci-dessus et $R_2$ est un radical de formule

$$-CH_2-\underset{\displaystyle CH_2Cl}{\underset{|}{C}}=CH_2$$

avec la N-monobenzhydrylpipérazine, la morpholine ou la pipéridine, pour obtenir un composé de formule générale I, dans laquelle $R_1$ est tel que défini ci-dessus et $R_2$ est un radical de formule générale (a) où $R_3$ est un groupe (4-benzhydryl-1-pipérazinyl)méthyle, 4-morpholinylméthyle ou 1-pipéridinylméthyle, et $R_4$ est un atome d'hydrogène, ou bien

B) on fait réagir une monoalkylpipérazine de formule générale II telle que définie ci-dessus avec un composé de formule générale IV

$$R_9NH_2 \qquad (IV)$$

dans laquelle $R_9$ est tel que défini dans la revendication 1, en présence de formaldéhyde, pour obtenir un composé de formule générale (I) dans laquelle $R_2$ est un radical de formule générale (f) tel que défini dans la revendication 1, et si on le désire, on hydrolyse le composé ainsi obtenu, en milieu acide, en un composé de formule générale (I) dans laquelle $R_2$ est un radical de formule (g) tel que défini dans la revendication 1, et si on le désire, on fait réagir le composé ainsi obtenu avec un acide carboxylique de formule générale V

$$R_9OH \qquad (V)$$

dans laquelle $R_9$ est tel que défini dans la revendication 1, pour obtenir un composé de formule générale (I), dans laquelle $R_2$ est un radical de formule générale (f), ou bien

C) on fait réagir une monoalkylpipérazine de formule générale (II) telle que définie ci-dessus avec un acide carboxylique de formule générale (VI)

$R_{10}COOH$

dans laquelle $R_{10}$ est tel que défini dans la revendication 1, pour obtenir un composé de formule générale (I) dans laquelle $R_2$ est un radical de formule générale (h) tel que défini dans la revendication 1, et, si on le désire, on convertit les composés pipérazines ainsi obtenus en leurs sels d'addition non toxiques.

3. Procédé conforme à la revendication 2, caractérisé en ce que les réactions selon A) et B) sont effectuées dans un alcanol en $C_{1-4}$, à des températures élevées, et en présence d'un carbonate ou d'un bicarbonate de métal alcalin ou alcalino-terreux, ou d'une amine tertiaire. .

4. Procédé conforme à la revendication 3, caractérisé en ce que la réaction est effectuée dans l'éthanol, à la température de reflux, en présence de bicarbonate de sodium ou de triéthylamine.

5. Procédé conforme à la revendication 2, caractérisé en ce que l'hydrolyse du composé de formule générale (I) dans laquelle $R_2$ est un radical de formule générale (f) est effectuée dans une solution aqueuse d'un mélange d'acide acétique et d'acide chlorhydrique à des températures élevées, de préférence à la température de reflux.

6. Procédé conforme à la revendication 2, caractérisé en ce que la réaction d'un composé de formule générale (I) dans laquelle $R_2$ est un radical de formule (g) avec un acide carboxylique de formule générale (V) est effectuée dans un solvant analogue à un éther, de préférence le tétrahydrofuranne, le N,N-diméthylformamide, ou un de leurs mélanges, en présence d'un catalyseur, de préférence le carbonyldiimidazole.

7. Procédé conforme à la revendication 2, caractérisé en ce que la réaction d'un composé de formule générale (II) avec un acide carboxylique de formule générale (VI) est effectuée dans un solvant analogue à un éther, de préférence le tétrahydrofuranne, le N,N-diméthylformamide, ou un de leurs mélanges, en présence d'un catalyseur, de préférence le carbonyldiimidazole.

8. Procédé conforme à la revendication 2, caractérisé en ce que la conversion des composés pipérazines en leurs sels d'addition non toxiques est effectuée dans un alcanol en $C_{1-4}$, de préférence le méthanol, l'éthanol, ou l'isopropanol.

9. Composition pharmaceutique comprenant an moins un composé conforme à la revendication 1, éventuellement en combinaison avec des véhicules et des adjuvants acceptables du point de vue pharmaceutique.